# EUROPEAN PATENT APPLICATION

(11) **EP 4 576 117 A1**
(43) Date of publication of application: **25.06.2025**
(21) Application number: 22955836.6
(22) Date of filing: 22.11.2022
(51) Int. Cl.: G16H 50/20, G16H 10/20, G16H 10/60, G16H 50/30, G16H 20/60, G16H 20/10, G06Q 50/00, G06Q 50/10, G06Q 20/14, G06Q 10/08

(54) **METHOD FOR SUPPORTING HEALTH CARE SERVICE, AND SYSTEM FOR SUPPORTING SAME**

(30) Priority: 19.08.2022 KR 20220103987
(71) Applicant: BTGin Co., Ltd., Daejeon 34024 (KR)
(72) Inventor: HOUR, Youl, Daejeon 34024 (KR)
(74) Representative: Jung, Minkyu
(86) International application number: PCT/KR2022/018520
(87) International publication number: WO 2024/038964

(57) **Abstract**

The present invention may be an invention that can search for/analyze a disease of interest and/or disease history of a user on the basis of questionnaire information and health records, and recommends customized health functional foods according to the search/analysis results. In addition, the present invention may be an invention that provides support to make it easy to receive posts, SNS posts, etc. registered by other members and share the received posts, SNS posts, etc. with acquaintances. In addition, the present invention may be an invention that can search for various information such as definitions/causes/related functionality/related foods/TIP/TALK, etc. about the disease of interest and supports a service in which location information about searched medical departments is displayed on an electronic map in order of proximity to a user through a location-based service and a search of related medical departments using keywords about age, cardinal symptoms, and accessory symptoms. **In** addition, the present invention may be an invention that searches and receives recommendations for disease names related to disease factors (nervous system, digestive/metabolic system, reproductive system, muscular system, sensory system, cardiovascular system, endocrine system, immune system, etc.), medicinal ingredients, medicines, types of health functional foods, sales information, etc., and supports purchase information (image, product name, consumer price, discount rate, selling price, quantity) about the searched/recommended health functional foods, and functions related to writing product reviews.

## Description

### Technical Field

The present invention relates to a system for supporting a health care service.

### Background Art

With the recent increase in life expectancy around the world, individuals are becoming increasingly interested in and aware of their health and illnesses.

The U-Health industry is gradually emerging as a self-healthcare sector that allows individuals to actively manage their health anytime, anywhere, in addition to the medical treatment they receive at a hospital.

In general, U-Health care can be categorized into the fields of biometric monitoring technology, daily life monitoring technology, and applied service technology, and with the recent development of smart terminals, sensors that can measure heart rate have begun to be installed, and based on the biometric monitoring technology of measuring heart rate, it has developed into a technology for acquiring signals from the human body, converting them into digital signals, and storing or transmitting them.

Healthcare platforms consisting of sensors that measure not only heart rate but also various vital signs, embedded systems that store digital data, personal computers, smartphones, and wearable devices are spreading to more diverse fields.

In this regard, a method for comprehensively providing health diagnosis, health supplement recommendation, and online product purchase has been researched and developed, and the prior art, Korean Laid Open Patent Publication No. 2002-0010293 (published on February 4, 2002), discloses a configuration that, by using a plurality of applications for receiving various healthcare-related information from a customer, extracting and hierarchically providing healthcare-related information to comprehensively provide healthcare services, it can support medical information search, diagnosis, medical appointment, comparative evaluation information on various products, information necessary for the management of specialized medical institutions, and online transactions, and it can perform basic health screenings with questions and answers and provide comparative evaluation data on items such as medicines, health foods, and medical devices, so that customers can easily make purchasing decisions on related items and manufacturers can use the information as marketing information.

However, even if the method is utilized, there is a risk that incorrect input data may lead to incorrect test results and corresponding health product recommendations because the information that can measure health is limited, and even if the user enters the information directly, it is not accurate unless the test results exist. In addition, since factors that affect health in addition to physical information are complex and affect each other, it is necessary to diversify and analyze each factor, but the method is a simple data input/output type of diagnosis, which significantly reduces the possibility of accurate diagnosis.

Therefore, it is necessary to develop a method that measures and inputs various factors that affect health together to perform accurate diagnosis and provide additional services based on them.

### Disclosure

### Technical Problem

An object of the present invention is to provide a system and method for supporting health care service, that may address the conventional issues.

### Technical Solution

A system supporting a health care service according to one embodiment of the present invention for solving the above problem comprises at least one mobile terminal receiving an online health questionnaire including basic information, lifestyle, physical symptoms and interests using a web page, app page, program or application related to a health care service, providing questionnaire answers to the online health questionnaire, and and receiving search and recommendation for health status results analyzed based on the questionnaire answers and types of health functional foods and nutritional ingredients suitable for or helpful thereto; and a health care service management server providing the online health questionnaire with question types consisting of basic information, lifestyle, symptoms and interests to the mobile terminal, collecting the questionnaire answers to the health questionnaire including single-choice, multi-choice entered on the mobile terminal, analyzing disease information and diseases according to lifestyle and symptoms included in the collected questionnaire answers, and recommending nutritional ingredients suitable for the analysis results and providing recommendation information and sales information on health functional foods containing the recommended nutritional ingredients.

A method supporting a health care service according to one embodiment of the present invention for solving the above problem comprises checking and providing, by a mobile terminal, health questionnaire answers to questions including basic information, lifestyle, physical symptoms provided by a health questionnaire platform; analyzing and providing, by a health care service management server, disease types and categories according to the user's health status based on the health questionnaire answers; recommending sales information and/or affiliated online shop sales information of preventive compositions effective for the disease types and categories analyzed by the health care service management server and health functional foods containing the preventive compositions; and requesting, by the user, purchase, payment, and delivery processing of health functional foods that the user searched for on the mobile terminal or received the recommendation from the health care service management server, requesting, by the health care service management server, when the product is delivered, a purchase review to be written to the mobile terminal, collecting the written purchase reviews and organizing the collected written purchase reviews into a database by member and product, and sharing the DB information with members.

### Advantageous Effects

The method and system supporting a health care service according to one embodiment of the present invention have the advantage of being able to search for/analyze a disease of interest and/or disease history of a user on the basis of questionnaire information and health records, and recommend customized health functional foods according to the search/analysis results. In addition,

Further, it easily receives posts, SNS posts, etc. registered by other members, shares the received posts, SNS posts, etc. with acquaintances, and searches for various information such as definitions/causes/related functionality/related foods/TIP/TALK, etc. about the disease of interest.

Further, it provides a service in which location information about searched medical departments is displayed on an electronic map in order of proximity to a user through a location-based service and a search of related medical departments using keywords about age, cardinal symptoms, and accessory symptoms.

In addition, it searches and receives recommendations for disease names related to disease factors (nervous system, digestive/metabolic system, reproductive system, muscular system, sensory system, cardiovascular system, endocrine system, immune system, etc.), medicinal ingredients, medicines, types of health functional foods, sales information, etc., and supports purchase information (image, product name, consumer price, discount rate, selling price, quantity) about the searched/recommended health functional foods, and functions related to writing product reviews to provide the advantage of allowing users to conveniently receive health management care services through mobile terminals.

### Description of Drawings

FIG. 1 is a network configuration diagram of a system supporting a health care service according to one embodiment of the present invention.
FIG. 2 is a detailed configuration diagram of the health care service management server illustrated in FIG. 1.
FIGS. 3 to 45 are exemplary views showing execution screens of a health care service platform executed on a mobile terminal illustrated in FIG. 1.
FIG. 46 is a flowchart illustrating a method for supporting a health care service according to an embodiment of the present invention.

### Mode for Invention

The Hereinafter, with reference to the accompanying drawings, embodiments of the present invention will be described in detail so that those skilled in the art can easily carry out the present invention. However, the present invention may be embodied in many different forms and is not limited to the embodiments described herein. In order to clearly describe the present invention in the drawings, parts irrelevant to the description are excluded, and similar reference numerals are assigned to similar parts throughout the specification.

Throughout the specification, when a part is said to be "connected" to another part, this includes not only the case of being "directly connected" but also the case of being "electrically connected" with another part in between. Further, it should be understood that when a part "includes" a certain component, this means that it may further include other components, not excluding other components, unless otherwise stated, and the presence or addition of one or more other features, numbers, steps, operations, components, parts, or combinations thereof is not precluded.

As used throughout the specification, the terms of degrees such as "about", "substantially", etc. are used in the meaning of a referenced value at or close to that value when inherent manufacturing and material tolerances are given, and they are used to prevent undue exploitation of the disclosure in which exact or absolute figures are recited to aid in the understanding of the present invention by unscrupulous infringers. The term "step of (doing)" or "step of" used throughout the specification of the present invention does not mean "step for."

In this specification, the term "unit" includes a unit realized by hardware, a unit realized by software, and a unit realized using both. Further, one unit may be realized using two or more hardware, and two or more units may be realized by one hardware.

In this specification, some of the operations or functions described as being performed by a terminal, apparatus, or device may be performed instead by a server connected to the terminal, apparatus, or device. Likewise, some of the operations or functions described as being performed by the server may also be performed by a terminal, apparatus, or device connected to the server.

In this specification, some of the operations or functions described as mapping or matching with the terminal may be interpreted as meaning mapping or matching with terminal's unique number or personal identification information, which is identifying data of a terminal.

Hereinafter, a system and method for supporting health care service according to an embodiment of the present invention will be described in more detail based on the accompanying drawings.

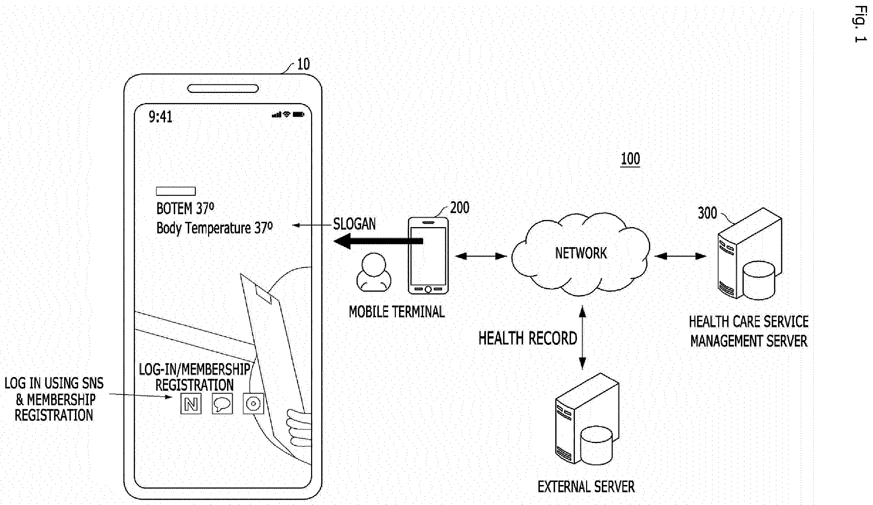

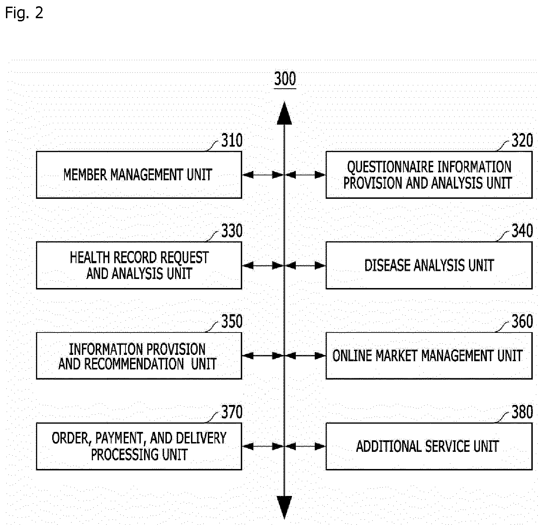

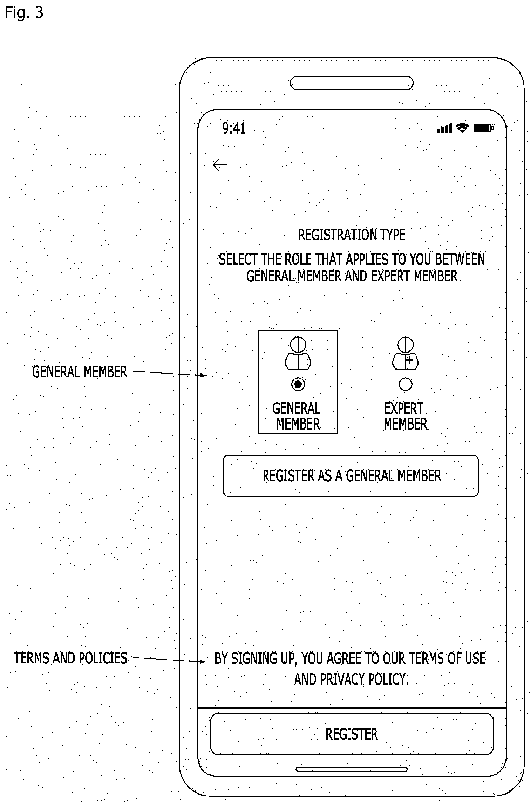

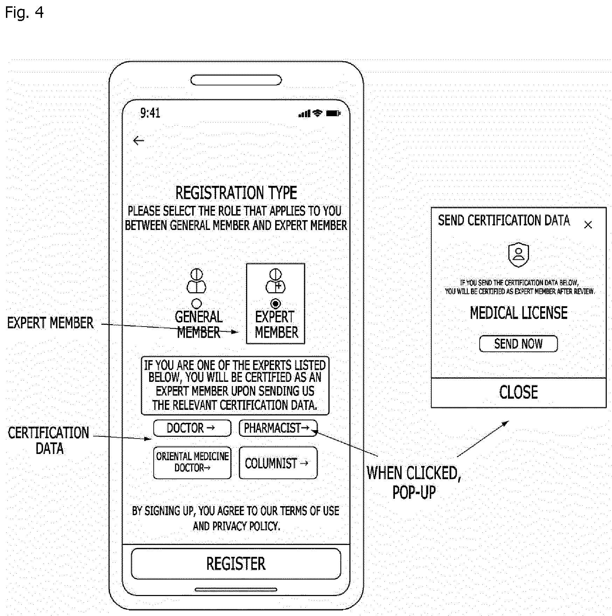

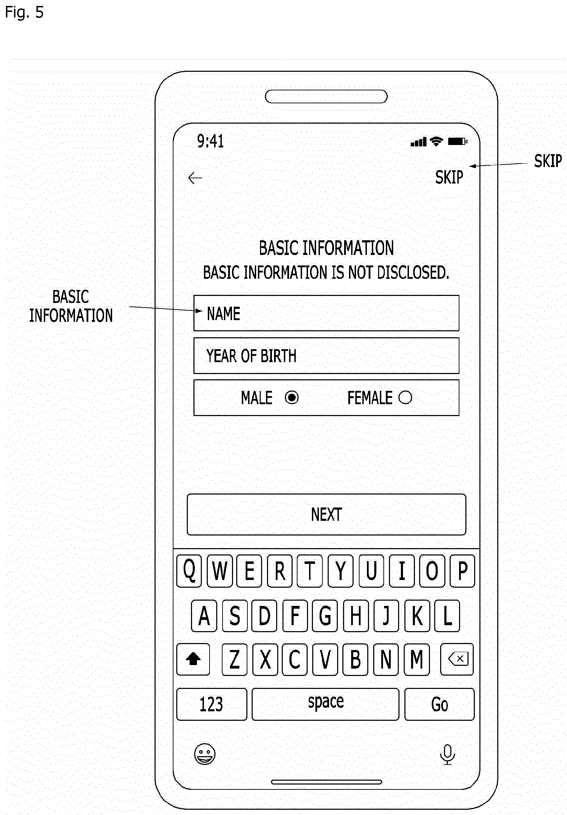

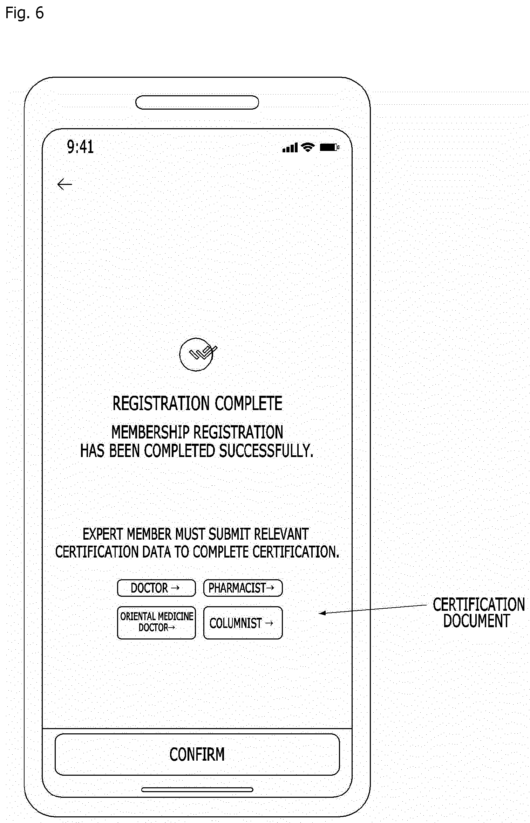

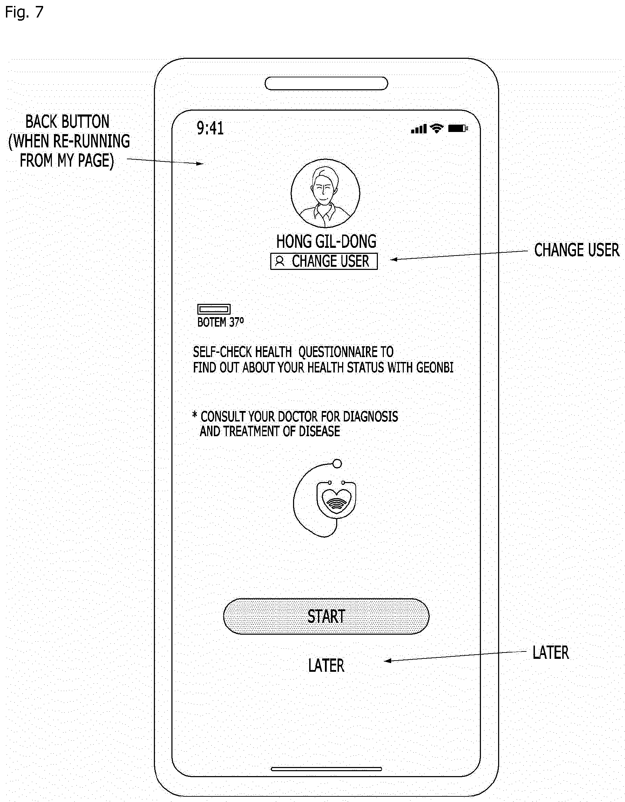

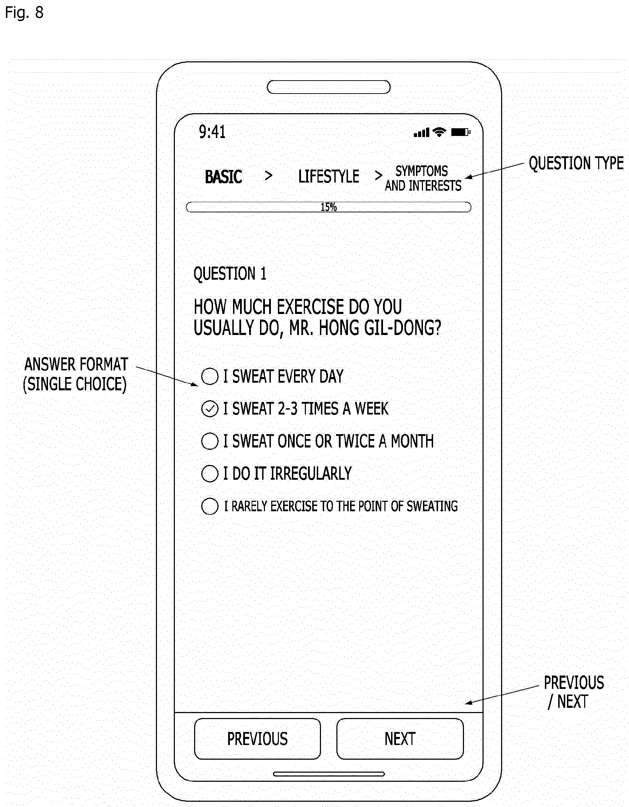

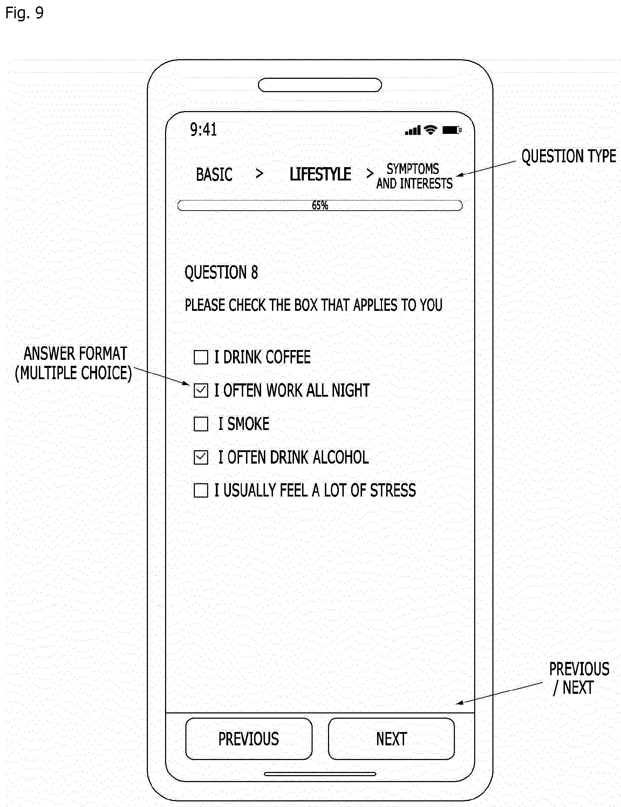

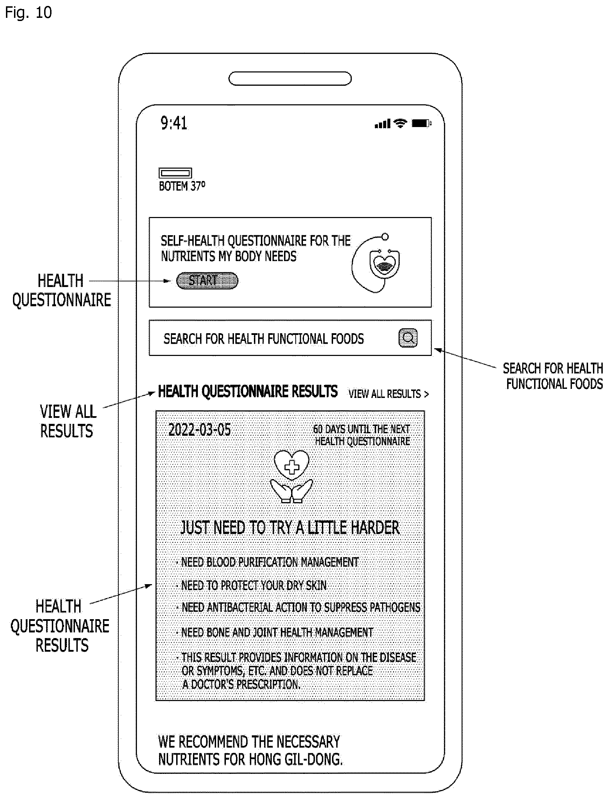

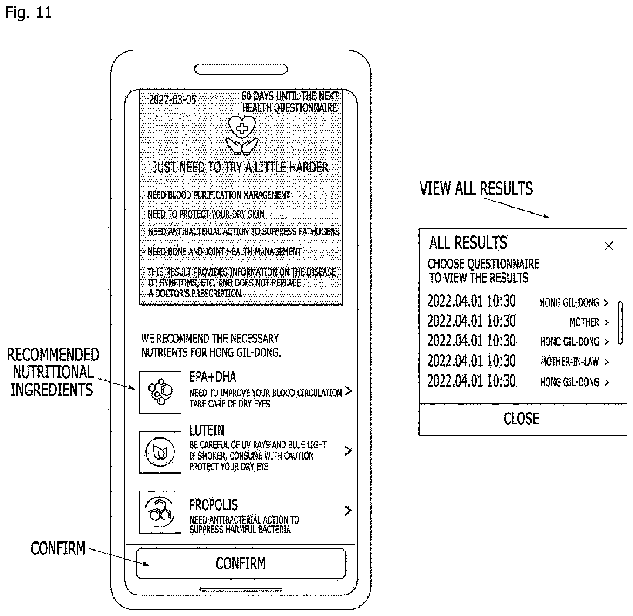

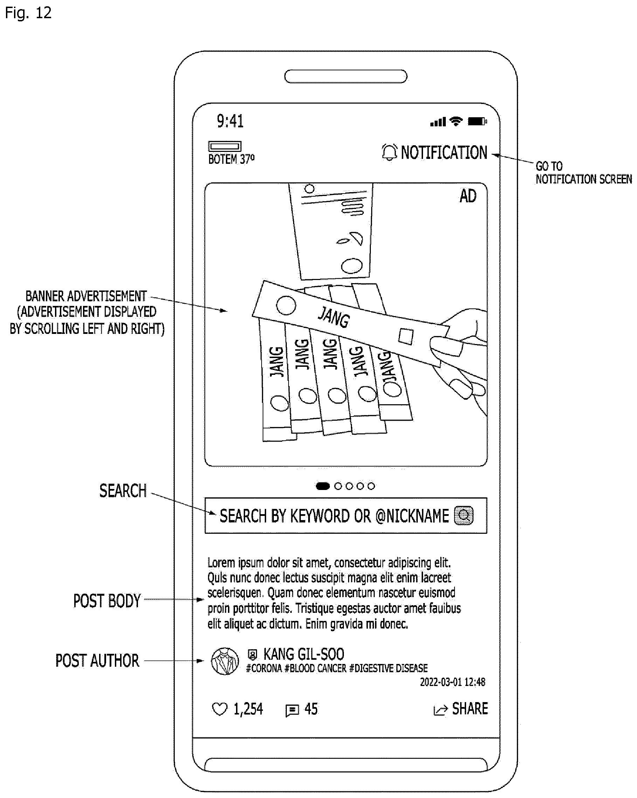

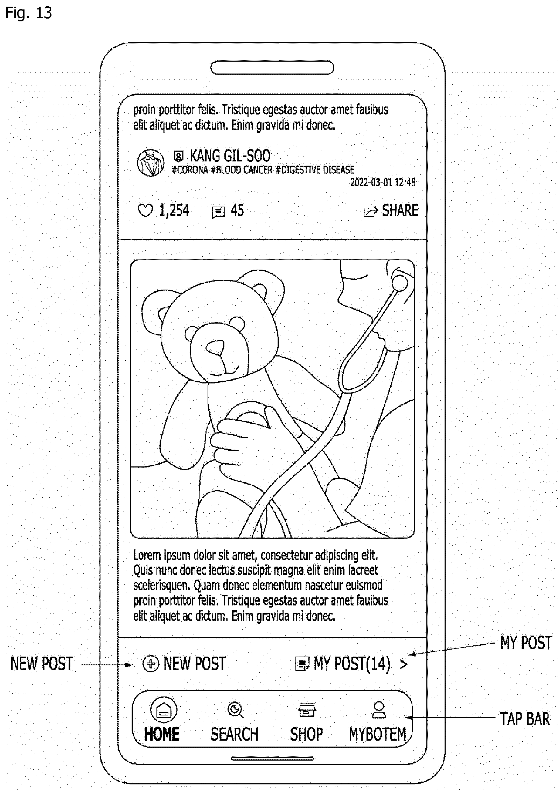

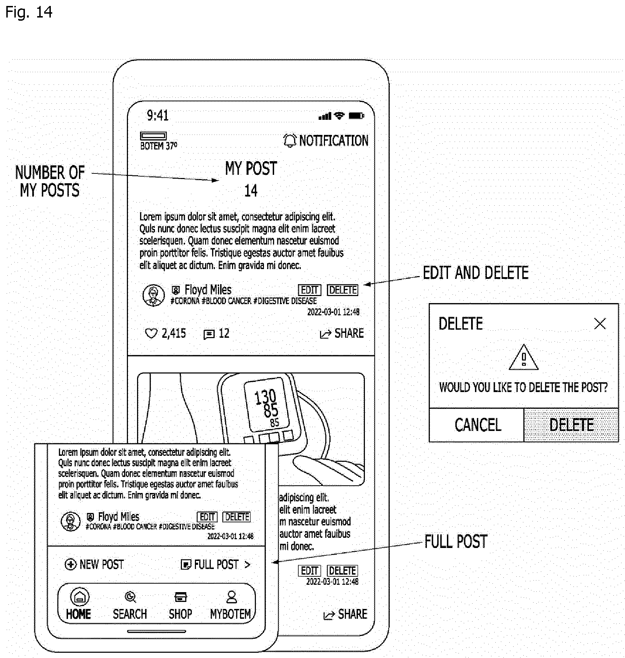

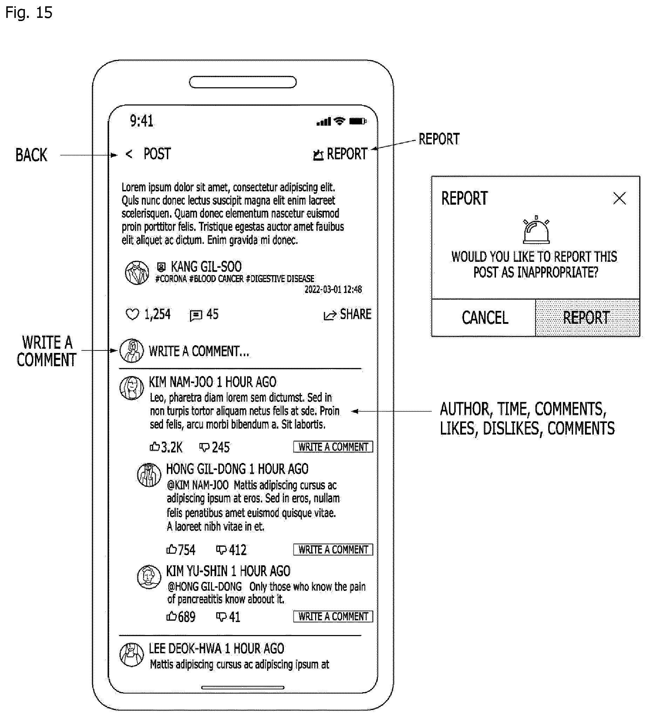

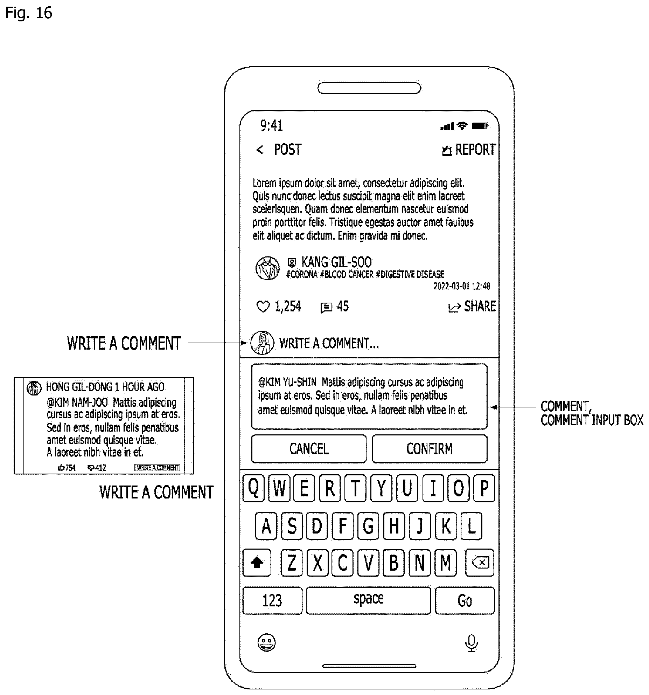

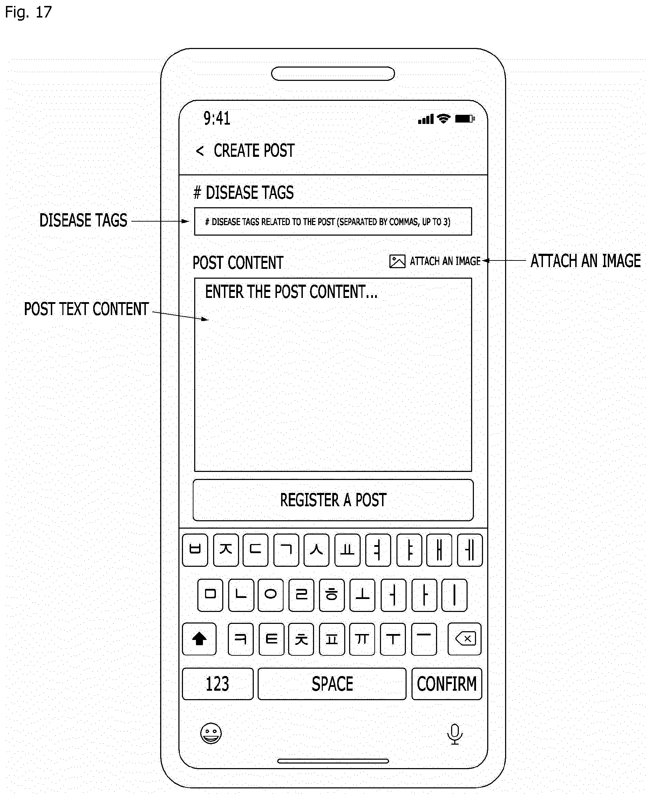

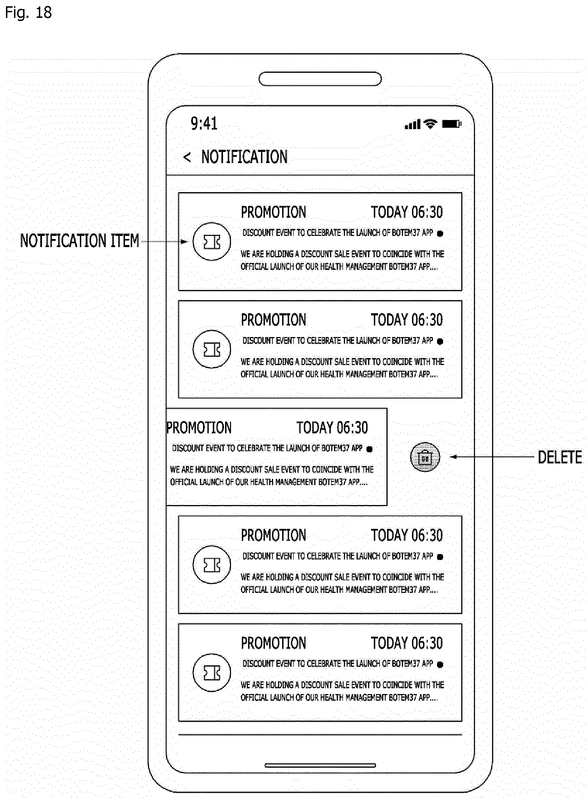

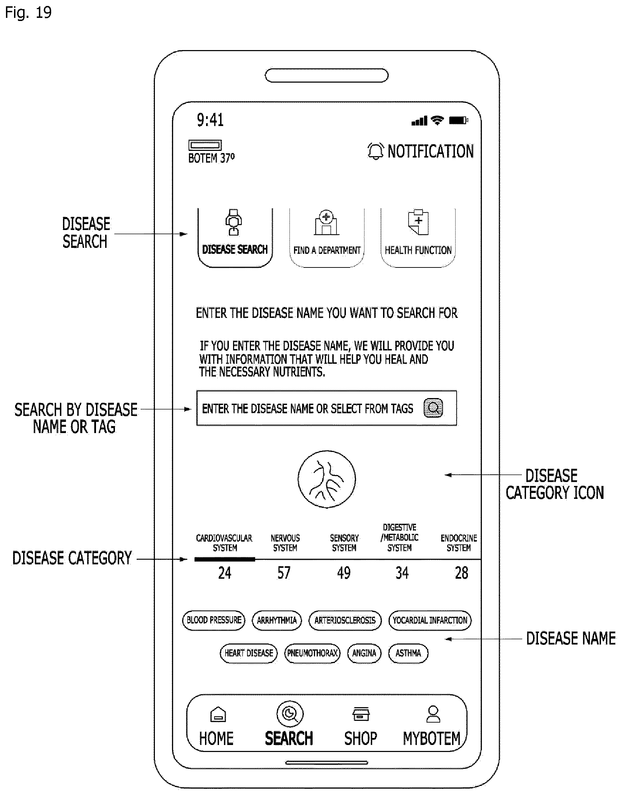

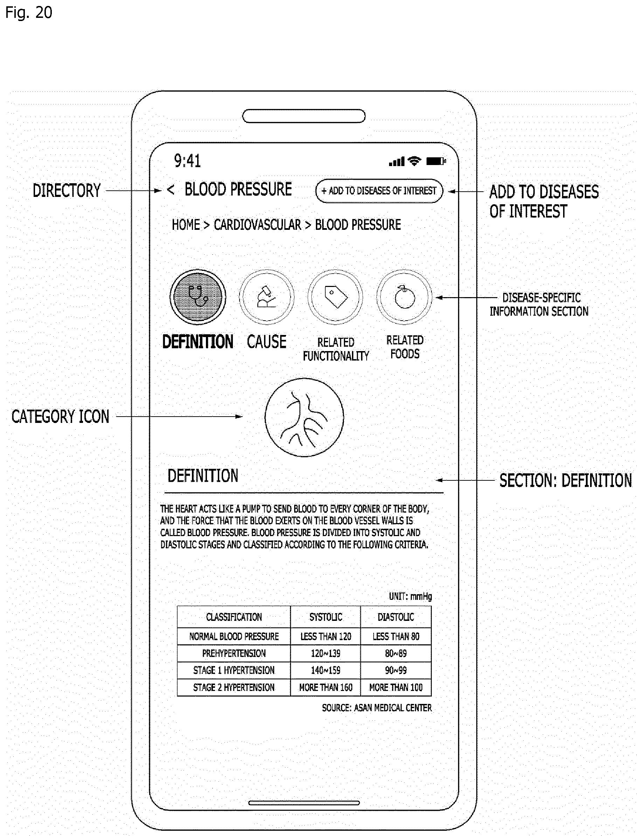

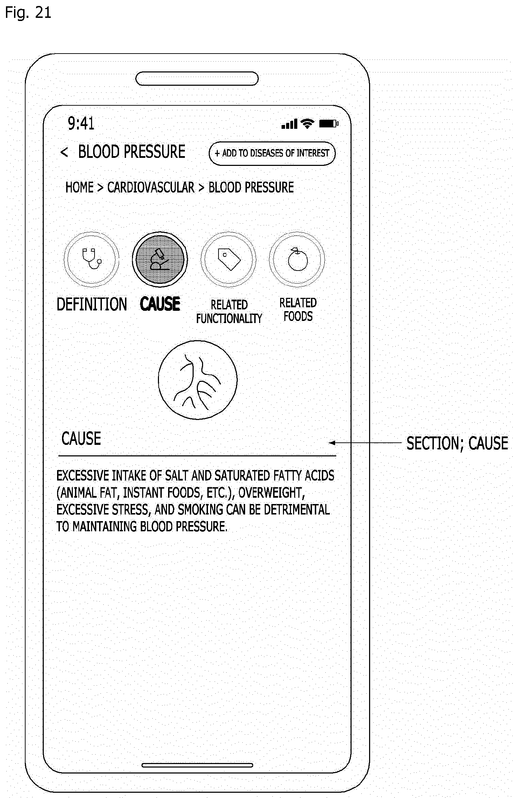

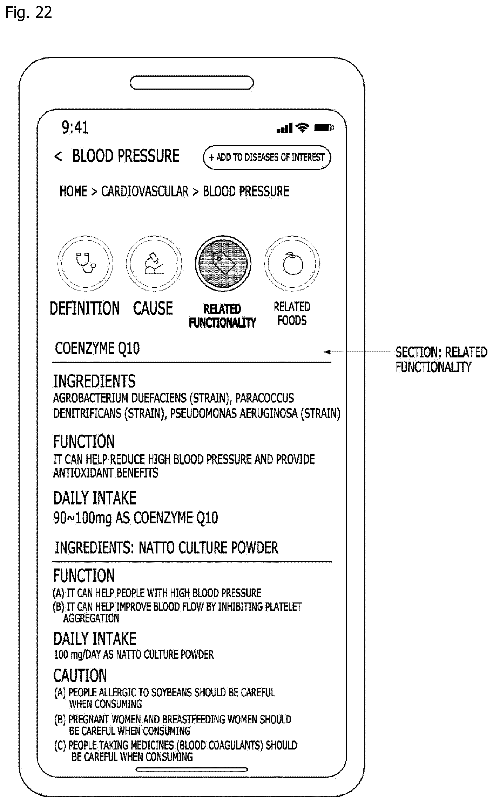

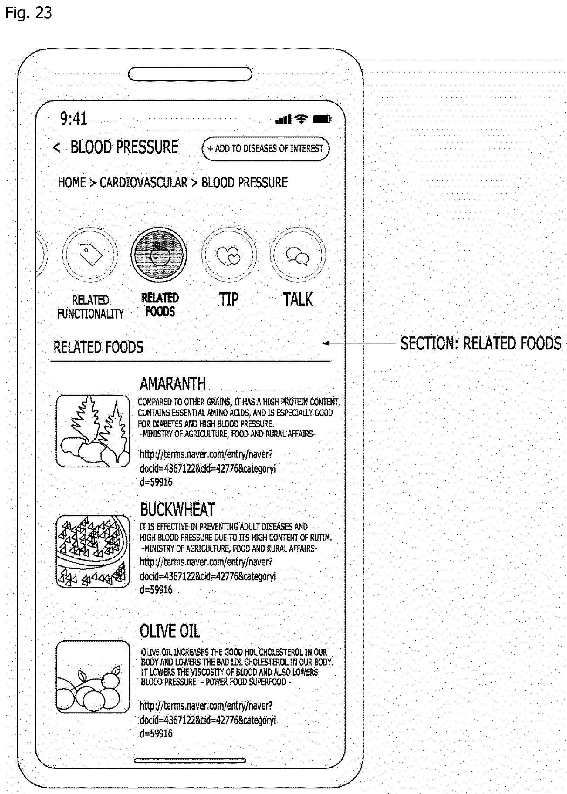

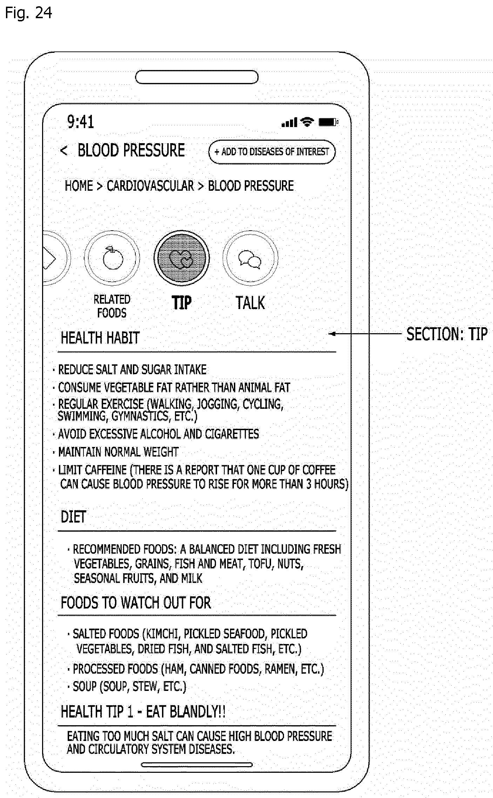

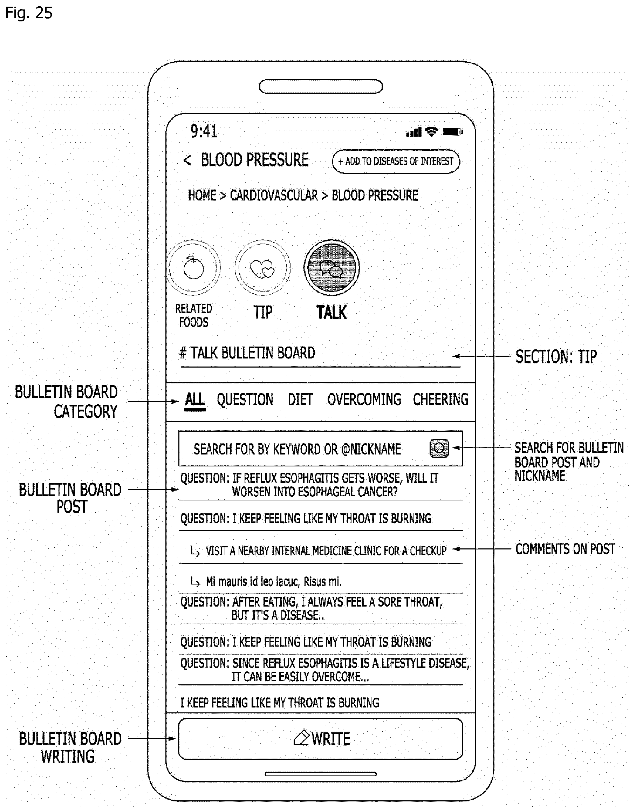

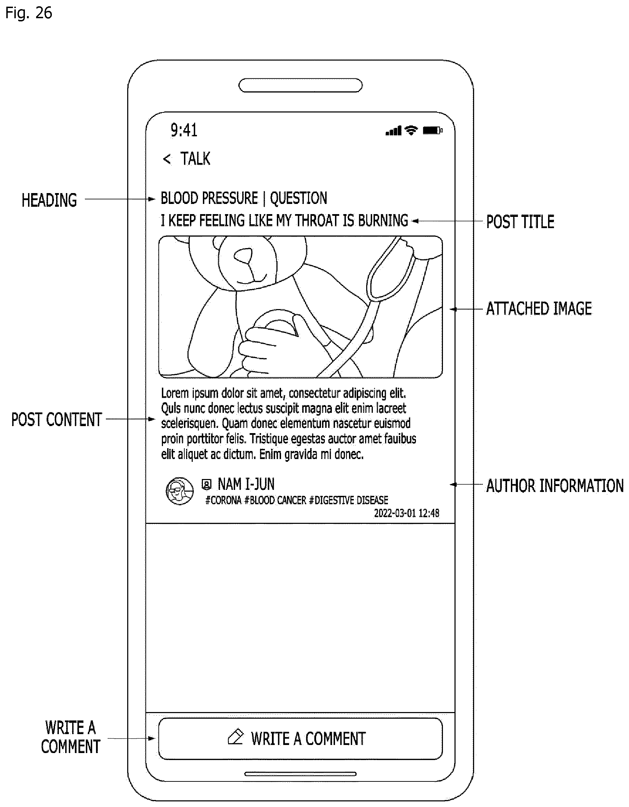

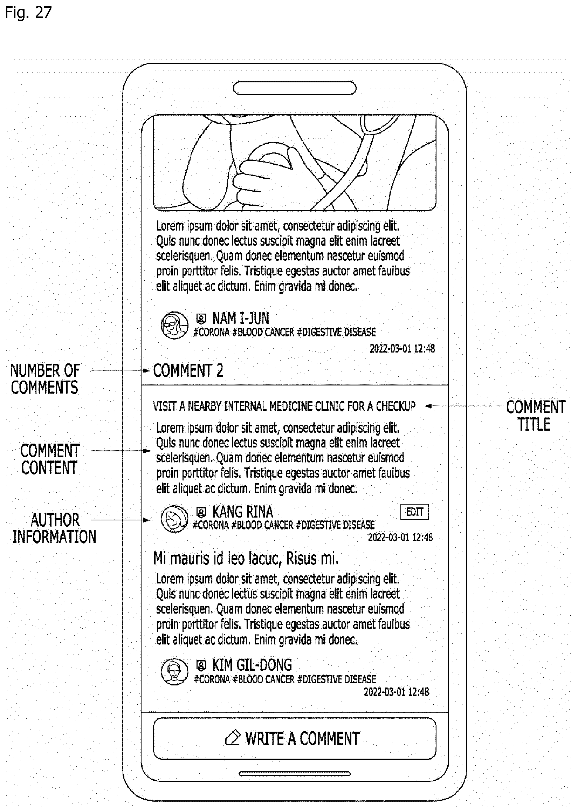

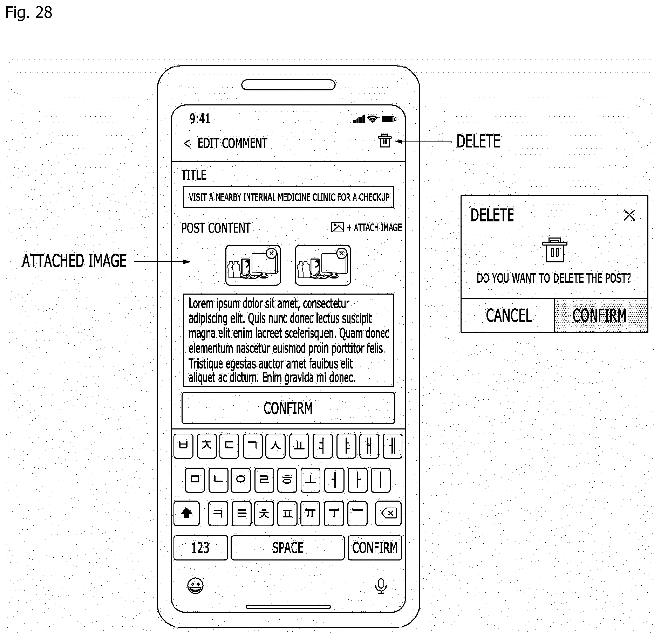

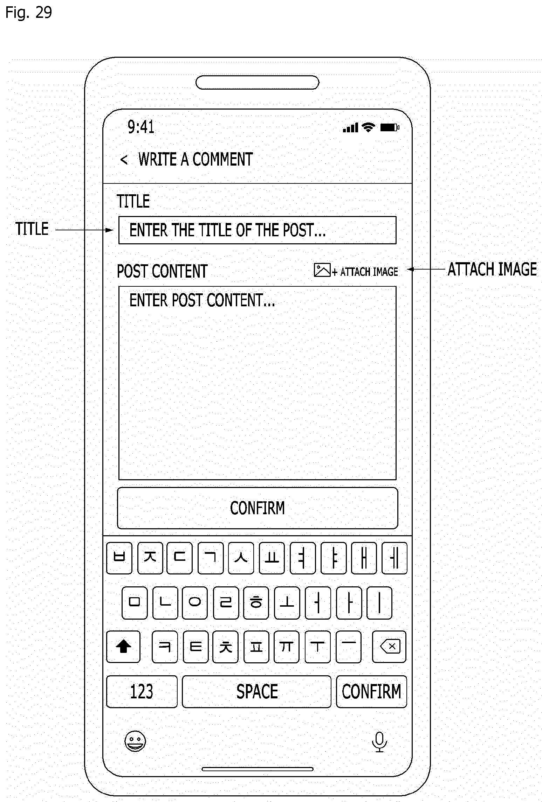

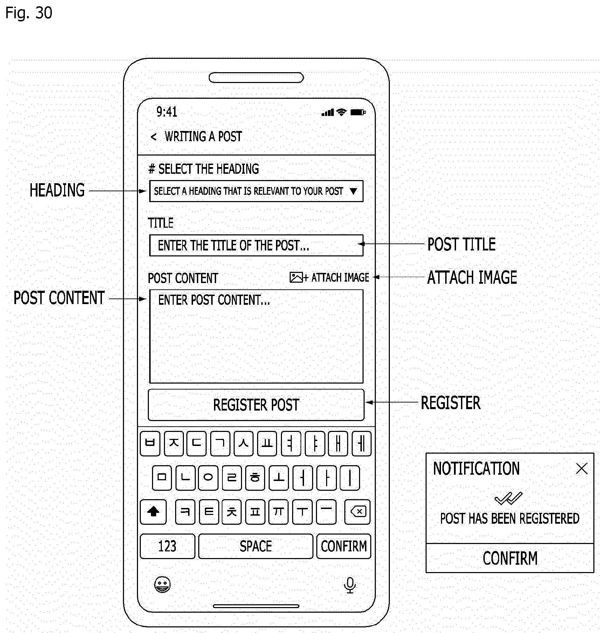

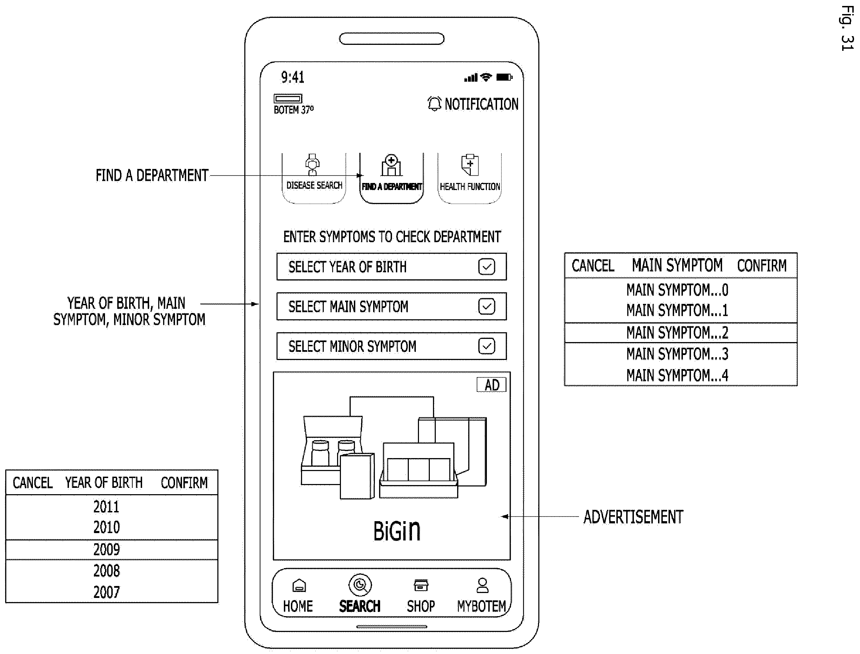

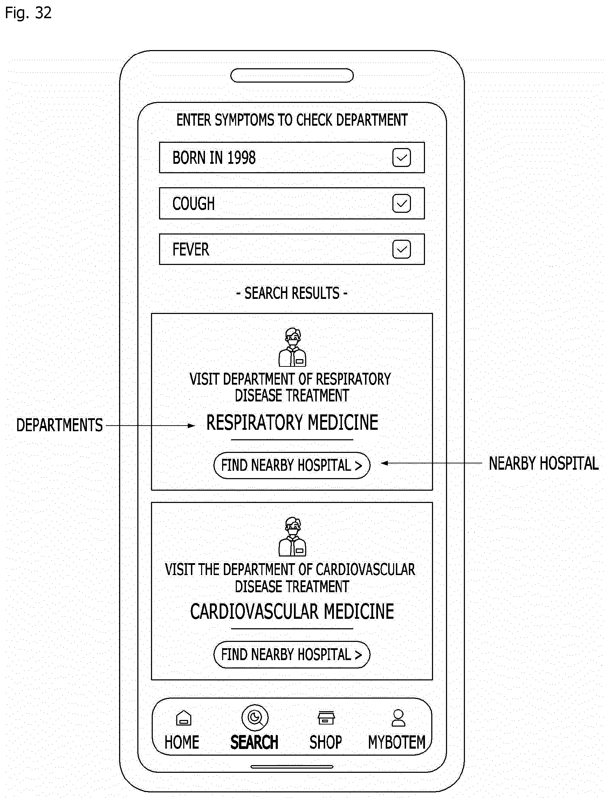

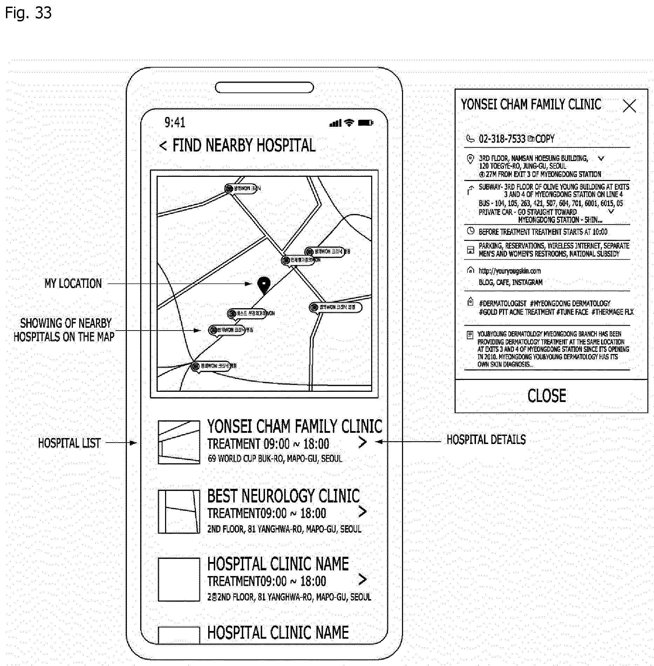

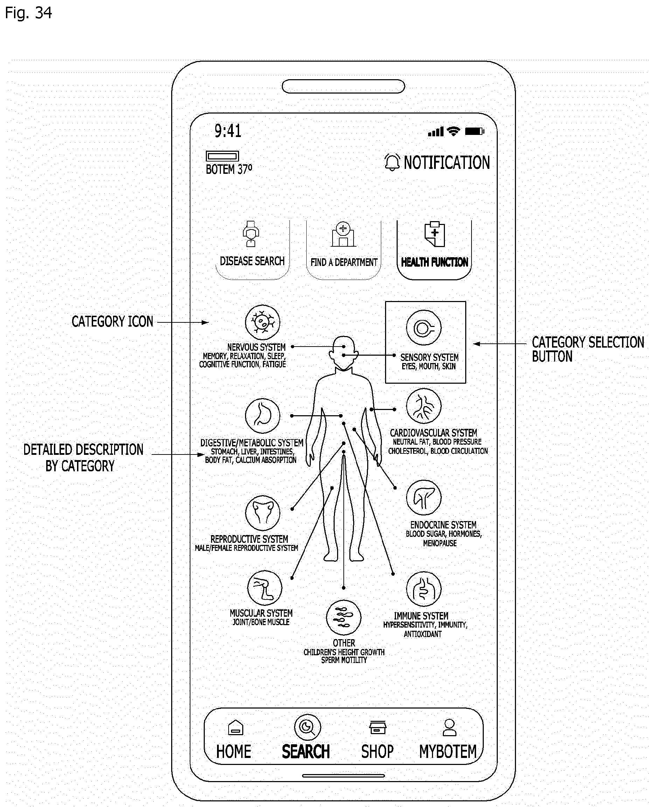

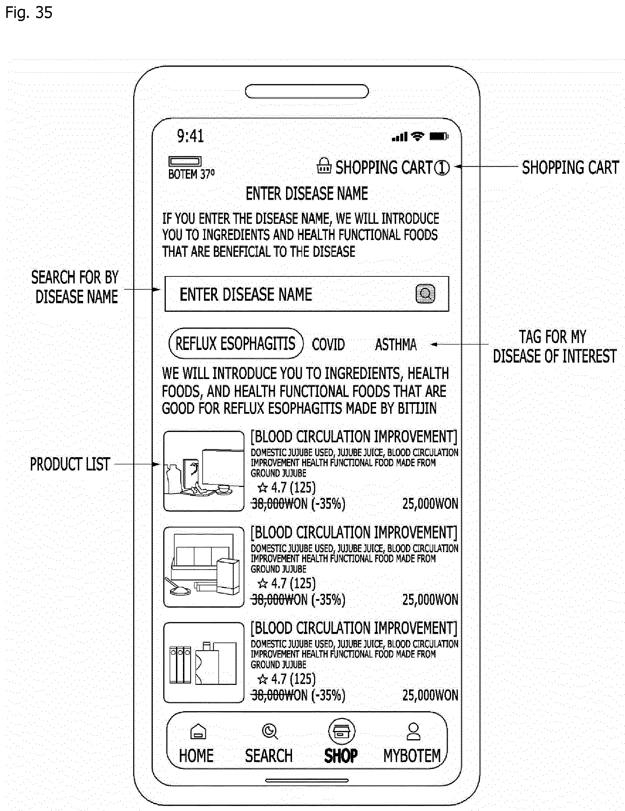

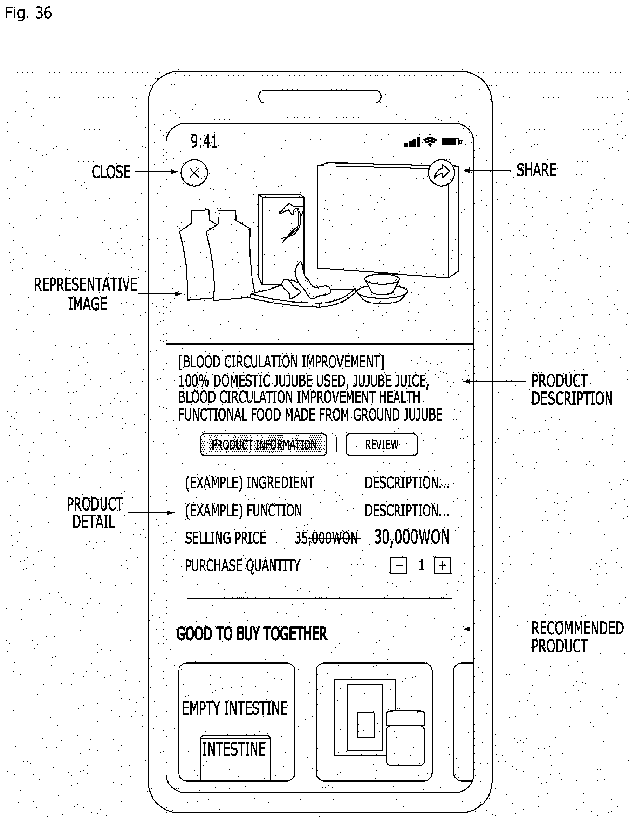

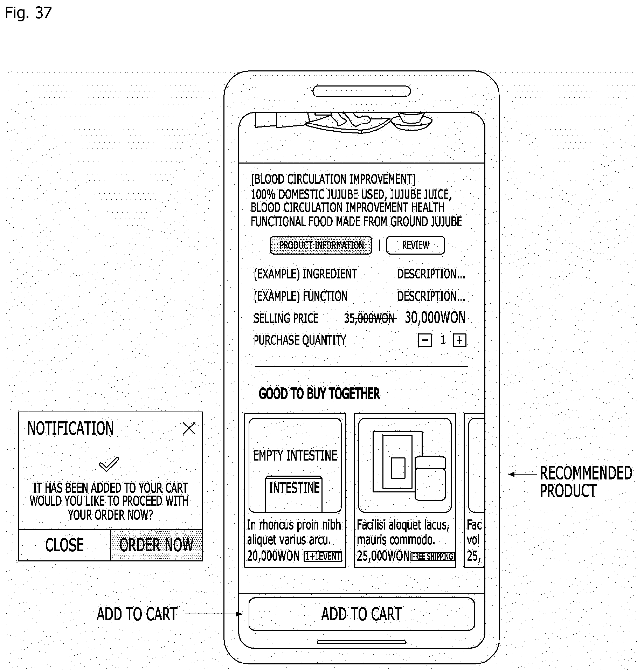

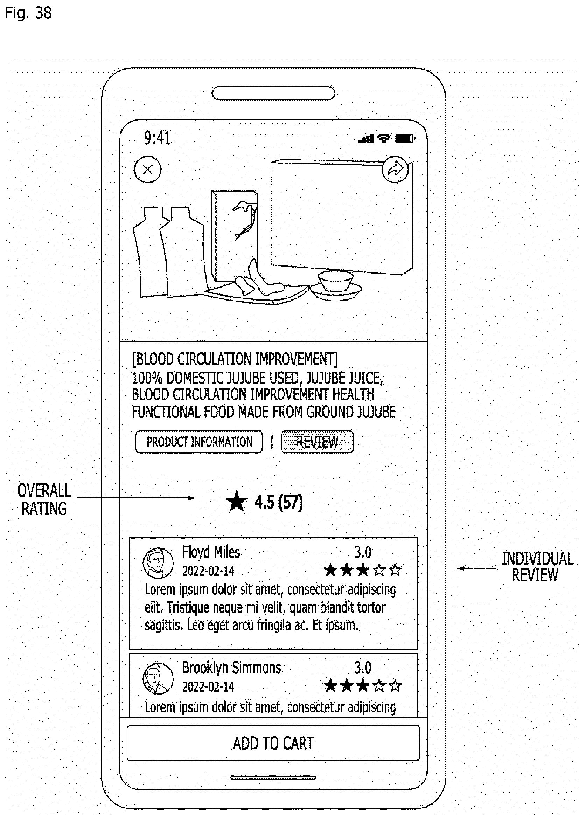

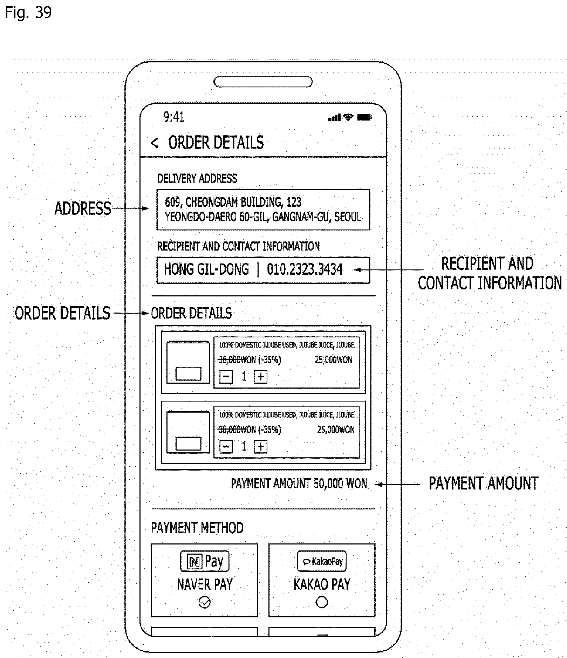

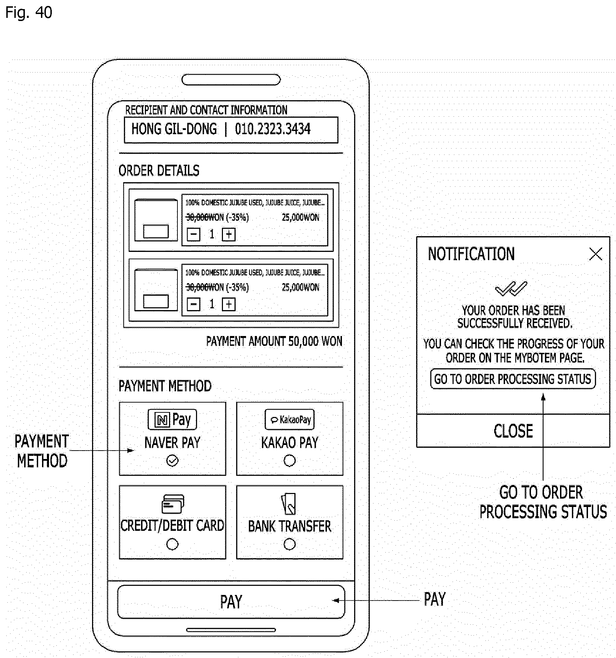

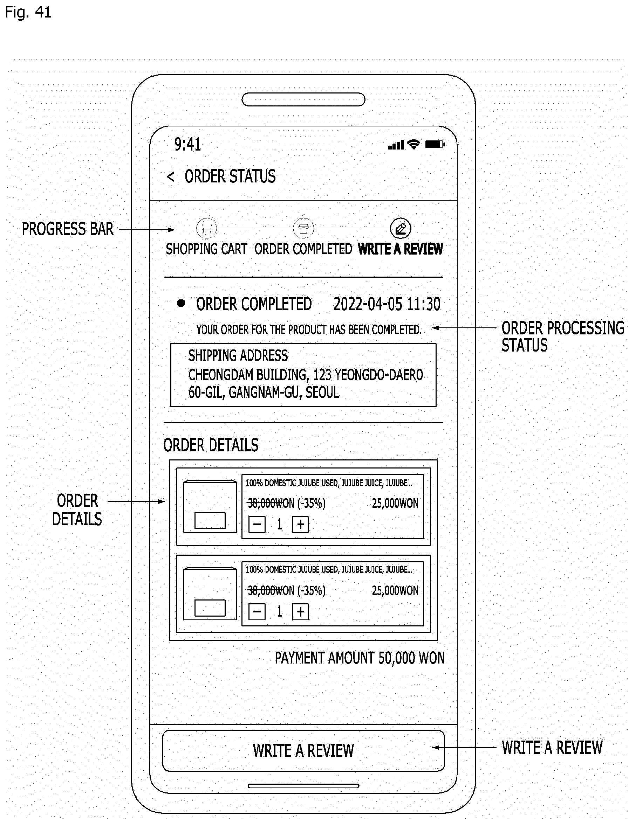

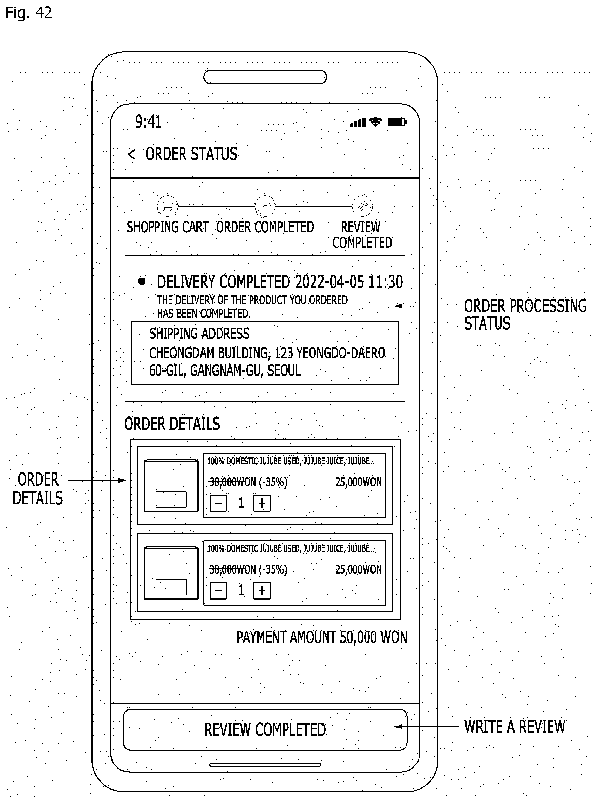

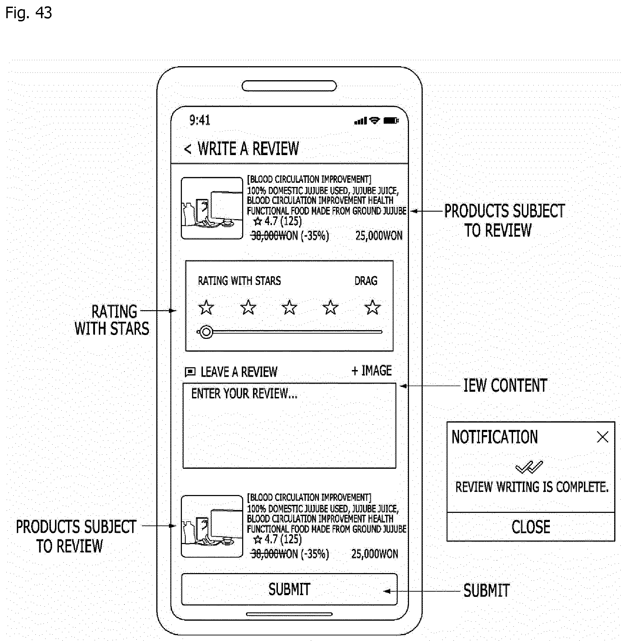

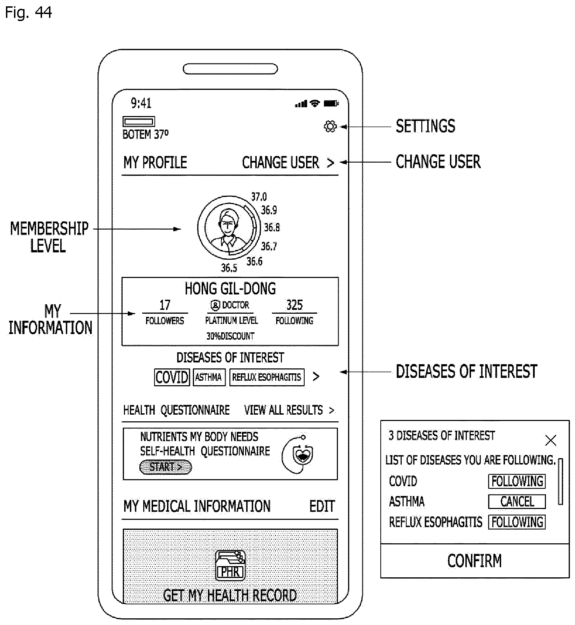

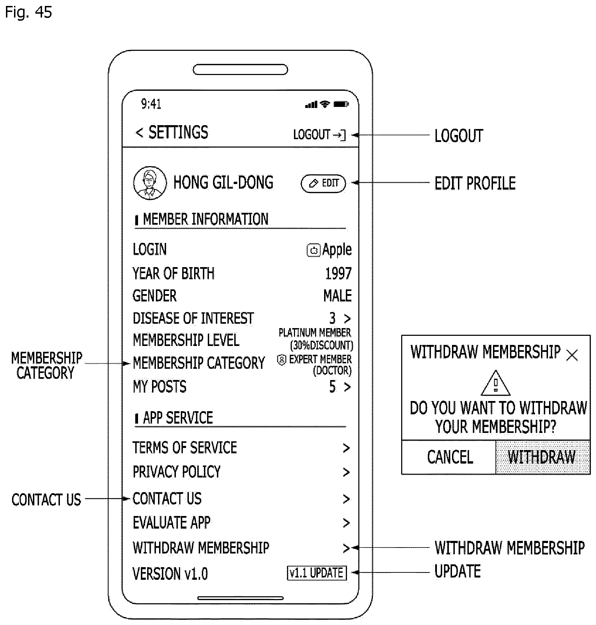

FIG. 1 is a network configuration diagram of a system supporting a health care service according to one embodiment of the present invention, FIG. 2 is a detailed configuration diagram of the health care service management server illustrated in FIG. 1, and FIGS. 3 to 45 are exemplary views showing execution screens of a health care service platform executed on a mobile terminal illustrated in FIG. 1.

First, as illustrated in FIG. 1, the system supporting a health care service (100) according to one embodiment of the present invention includes a mobile terminal (200) and a health care service management server (300).

Each component communicates over a network in which the network refers to a connection structure capable of exchanging information between nodes such as a plurality of terminals and servers. Examples of such networks include radio frequency (RF), 3rd generation partnership project (3GPP) network, long term Evolution (LTE) network, 5th generation partnership project (5GPP) network, world interoperability for microwave access (WIMAX) network, Internet, local area network (LAN), wireless local area network (wireless LAN), wide area network (WAN), personal area network (PAN), Bluetooth network, near field communication (NFC) network, satellite broadcasting network, analog broadcasting network, digital multimedia broadcasting (DMB) network, etc., but are not limited thereto.

It will be clear that in the following, the term "at least one" is defined as a term including singular and plural, and even if the term "at least one" does not exist, each component may be present in singular or plural, and it can mean singular or plural. Further, each component is provided in singular or plural. which can be changed according to the embodiment.

The mobile terminal (200) may be a configuration receiving a health questionnaire including basic information, lifestyle, physical symptoms and interests using a web page, app page, program or application related to a health care service, providing questionnaire answers thereto, and and receiving search and recommendation for health status results analyzed based on the questionnaire answers and types of health functional foods and nutritional ingredients suitable for or helpful thereto.

Additionally, it may be a terminal that receives pop-up information on a list of all health questionnaires completed hereinbefore.

In addition, the mobile terminal (200) may be a terminal that receives posts, SNS posts, etc. registered by other members using a web page, app page, program or application related to a health care service, and shares the received posts, SNS posts, etc. with acquaintances.

In addition, the mobile terminal (200) may be a terminal that supports a search tab function for diseases, departments, and health functions using a web page, app page, program or application related to a health care service, and shares the received posts, SNS posts, etc. with acquaintances.

Here, the disease search tab supports the function of searching for information on definition/cause/related functionality/related foods/TIP/TALK, and the department search tab supports the function of searching for related departments by keywords such as age, main symptom, and side symptom, and supports the function of displaying the departments searched for in order of proximity to the user on an electronic map through a location-based service.

Next, the health function tab supports the function of searching and recommending disease names related to disease factors (nervous system, digestive/metabolic system, reproductive system, muscular system, sensory system, cardiovascular system, endocrine system, immune system, etc.), medicinal ingredients, medicines, types of health functional foods, sales information, etc., and supports purchase information (image, product name, cost, discount rate, sales price, quantity) of purchased health functional foods and a function for writing product reviews.

Meanwhile, the mobile terminal (200) supports the function of displaying the medication information, medical history, and vaccination records by receiving the user's health record information from the National Health Insurance Service under the Ministry of Health and Welfare.

At this time, it may be a terminal that receives the function of displaying, based on the medication information, medical history, and vaccination, at least one disease of interest information related thereto, setting and registering diseases of interest and recommends health functional foods.

The mobile terminal (200) may be implemented as a computer capable of accessing a remote server or terminal through a network. Here, the computer may include, for example, a laptop computer or a desktop computer equipped with a navigation system and a web browser. In this case, at least one user terminal (100) may be implemented as a terminal capable of accessing a remote server or terminal through a network. The at least one user terminal (100) may for example, include all kinds of handheld-based wireless communication devices such as navigation device, personal communication system (PCS), global system for mobile communications (GSM), personal digital cellular (PDC), personal handphone system (PHS), personal digital assistant (PDA), international mobile telecommunication (IMT)-2000, code division multiple access (CDMA)-2000, W-code division multiple access (W-CDMA), wireless broadband internet (WiBro) terminal, smartphone, smart pad, and tablet PC. as a wireless communication device that ensures portability and mobility,

Next, the health care service management server (300) may be a server that provides a health care service web page, app page, program or application.

In addition, the health care service management server (300) may be a server that receives membership registration as either general or expert from at least one mobile terminal (200), or transmits basic questionnaire results to the mobile terminal (200) when responding to a questionnaire without receiving membership registration. For reference, when registering as an expert member, if the member provides certification documents (medical license or certificate) that prove that he or she is an expert (doctor, pharmacist, oriental medicine doctor, columnist, etc.), the member may be registered as an expert member after going through an expert review.

Further, the health care service management server (300) may be a server that provides online health questionnaire information with question types consisting of basic information, lifestyle, and symptoms and interests to the mobile terminal (200), collects health questionnaire answers for health questionnaires (single-choice, multi-choice) entered in the mobile terminal (200), analyzes disease information and diseases according to lifestyle and symptoms included in the health questionnaire answers, and provides recommendations for nutritional ingredients suitable for this and recommendation information for health functional foods containing the recommended nutritional ingredients.

**In** addition, the health care service management server (300) may be a server that provides an interface that supports functions such as searching/writing/editing/deleting health-related posts and SNS posts written by general and expert members and a comment function for posts and SNS posts, and that provides an interface that supports functions such as sharing posts and SNS posts searched or written by general and expert members with other members.

Additionally, the health care service management server (300) may be a server that provides an interface that supports a search tab function for diseases, departments, and health functions.

Here, the disease search tab supports the function of searching for information on definition/cause/related functionality/related foods/TIP/TALK, and the department search tab supports the function of searching for related departments by keywords such as age, main symptom, and side symptom, and supports the function of displaying at least one departments searched for in order of proximity to the user on an electronic map through a location-based service.

Next, the health function tab supports the function of searching and recommending detailed disease names related to disease factors (nervous system, digestive/metabolic system, reproductive system, muscular system, sensory system, cardiovascular system, endocrine system, immune system, etc.) (e.g., if cardiovascular system is selected, blood pressure/arrhythmia/arteriosclerosis/myocardial infarction/heart disease/pneumothorax/angina/asthma), nutrients, pharmaceutical ingredients, medicines, types of health functional foods containing nutrients to prevent or treat the searched disease, health habit information, sales information, etc., and supports the function of checking the purchase/payment information (image, product name, consumer price, discount rate, sales price, quantity, payment amount) of the purchased health functional food and writing a product review.

Further, the health care service management server (300) may be a server that receives the user's health record information from the National Health Insurance Service under the Ministry of Health and Welfare, classifies the medication information, medical history, and vaccination in the user's health record information, and analyzes and provides, based on the medication information, medical history, and vaccination, at least one disease of interest information related thereto.

More specifically, the health care service management server (300) includes a member management unit (310), a questionnaire information provision and analysis unit (320), a health record request and analysis unit (330), a disease analysis unit (340), an information provision unit (350), an online market management unit (360), and an additional service unit (370).

The member management unit (310) is configured to manage member information using the health care service platform, and provides information on the guidance procedures required for membership registration.

In addition, the member management unit (310) may be configured to request a mobile terminal to select either a general member or an expert member when registering for membership, and to proceed with membership registration using personal basic information (membership registration with gender, name, and year of birth) to prevent exposure of personal information.

In addition, the member management unit (310) may be configured to request registration of documents (e.g., medical license, etc.) for certifying an expert member as mandatory information in the case of an expert member. Additionally, it may be configured to request a judgment on whether an external certification document is forged or request a judgment from a group of experts.

Next, the questionnaire information provision and analysis unit (320) may be configured to provide online health questionnaire information having question types consisting of basic information, lifestyle, and symptoms and interests, collect health questionnaire answers for health questionnaires (single-choice, multi-choice) entered on a mobile terminal (200), and analyze and provide health questionnaire results included in the health questionnaire answers.

Next, the health record request and analysis unit (330) may be configured to be linked with an external server (e.g., the Ministry of Health and Welfare) to request health record information of a member based on the identity authentication information presented by the mobile terminal (200), and mediate health record information transmitted from the external server to the mobile terminal.

Further, the health record request and analysis unit (330) may be configured to extract and analyze medication information, medical history, and vaccination records from the provided health record information, and provide them to the mobile terminal.

Next, the disease analysis unit (340) may be configured to analyze the presence or absence of a disease, disease incidence rate, disease factors, disease types, etc. based on information on medication information, medical history, and vaccination records analyzed by the questionnaire information provision and analysis unit (320) and/or health record request and analysis unit (330).

For reference, disease factors include the nervous system, digestive/metabolic system, reproductive system, muscular system, sensory system, cardiovascular system, endocrine system, immune system, etc., and the disease type may correspond to a detailed component of the disease factor.

Next, the information provision and recommendation unit (350) may be configured to provide and recommend information on the types, dosage methods, TIPs, daily intake, etc. of nutritional compositions and health functional foods containing nutritional compositions for preventing or suppressing diseases analyzed by the disease analysis unit (340).

Furthermore, it may be configured to provide and recommend information on pharmaceutical ingredients, medicines, etc. for suppressing or treating the analyzed diseases.

Next, the online market management unit (360) may be configured to register and manage online retailers that sell affiliated online functional foods and products sold from the online retailers. The online market management unit (360) may be linked with the information provision and recommendation unit (350).

Next, the order, payment, and delivery processing unit (370) may be configured to provide and process a series of information on order details, payment (payment method), and delivery for health functional foods searched and purchased or recommended and ordered on the mobile terminal (200).

Next, the additional service department (380) may be configured to provide an interface that supports searching/writing/editing/deleting functions for health-related posts and SNS posts written by general and expert members and a comment function for posts and SNS posts.

In addition, it may be configured to provide an interface that supports a function for sharing posts and SNS posts searched or written by general and expert members with other members.

Further, it may be configured to support search tab functions for diseases, departments, and health functions, and here, the disease search tab may be configured to support the function of searching for information on definition/cause/related functionality/related foods/TIP/TALK, and
the department search tab may be configured to support the function of searching for related departments by keywords such as age, main symptom, and side symptom, and support the function of displaying at least one departments searched for in order of proximity to the user on an electronic map through a location-based service.

Further, it may be configured to support purchase information (image, product name, consumer price, discount rate, sales price, quantity) of purchased health functional foods and a product review writing function.

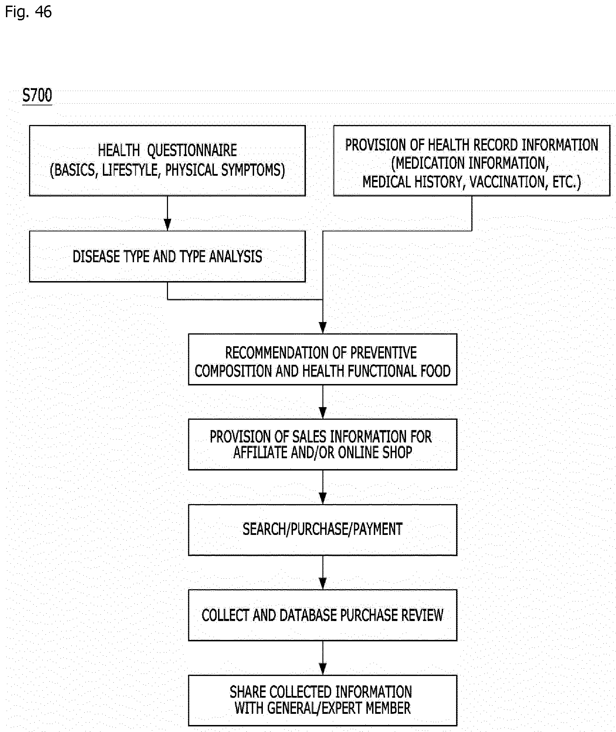

FIG. 46 is a flowchart illustrating a method for supporting a health care service according to an embodiment of the present invention.

As shown in FIG. 46, in the method for supporting a health care service (S700) according to one embodiment of the present invention, a mobile terminal (200) checks and provides health questionnaire answers to questions (basic information, lifestyle, physical symptoms) in a health questionnaire provided by a health care service platform, and then a health management care service management server (300) analyzes and provides the type and category of questions according to the user's health status based on the health questionnaire answers.

Then, the health care service management server (300) recommends sales information and/or affiliated online shop sales information of preventive compositions effective for the disease types and categories analyzed and health functional foods containing the preventive compositions.

Thereafter, the method includes a process in which a user searches for or requests purchase/payment/delivery processing of a recommended health functional food on the mobile terminal (200), and when the product is delivered, the health care service management server (300) requests the mobile terminal (200) to write a purchase review, collects the written purchase reviews and creates a database for each member and product, and shares the DB information with members.

Therefore, the method and system supporting a health care service according to one embodiment of the present invention have the advantage of being able to search for/analyze a disease of interest and/or disease history of a user on the basis of questionnaire information and health records, and recommend customized health functional foods according to the search/analysis results.

Further, it is easy to receive posts, SNS posts, etc. registered by other members and share the received posts, SNS posts, etc. with acquaintances.

Further, it can search for various information such as definitions/causes/related functionality/related foods/TIP/TALK, etc. about the disease of interest and receive a service in which location information about searched medical departments is displayed on an electronic map in order of proximity to a user through a location-based service and a search of related medical departments using keywords about age, cardinal symptoms, and accessory symptoms.

In addition, it searches and receives recommendations for disease names related to disease factors (nervous system, digestive/metabolic system, reproductive system, muscular system, sensory system, cardiovascular system, endocrine system, immune system, etc.), medicinal ingredients, medicines, types of health functional foods, sales information, etc., and supports purchase information (image, product name, consumer price, discount rate, selling price, quantity) about the searched/recommended health functional foods, and functions related to writing product reviews to provide the advantage of allowing users to conveniently receive health management care services through mobile terminals.

The method according to the embodiment may also be implemented in the form of a recording medium containing computer-executable instructions, such as an application or program module executed by a computer. The computer-readable medium may be any available medium that can be accessed by a computer, and includes both volatile and nonvolatile media, removable and non-removable media. Further, the computer-readable medium may include all computer storage media. The computer storage media includes both volatile and nonvolatile, removable and non-removable media implemented by any method or technology for storing information, such as computer-readable instructions, data structures, program modules, or other data.

The method according to the embodiment of the present invention may be executed by an application basically installed on a terminal (which may include a program included in a platform or operating system basically installed on the terminal), and may also be executed by an application (i.e., a program) directly installed on a master terminal by a user through an application management server, such as an application store server, an application, or a web server related to the corresponding service. In this sense, the method according to the embodiment of the present invention may be implemented as an application (i.e., a program) that is installed basically on a terminal or directly installed by a user, and may be recorded on a computer-readable recording medium such as a terminal.

It will be understood that the description of the present invention is for illustrative purposes, and those skilled in the art to which the present invention belongs will be able to understand that it can be easily modified into other specific forms without changing the technical spirit or essential features of the present invention. Therefore, the embodiments described above should be understood as illustrative in all respects and not limiting. For example, each component described as a single type may be implemented in a distributed manner, and similarly, components described as distributed may be implemented in a combined form.

It should be interpreted that the scope of the present invention is indicated by the claims to be described later rather than the detailed description above, and all changes or modifications derived from the meaning and scope of the claims and equivalent concepts thereof are included in the scope of the present invention.

### [Description of reference numbers]

100: system for supporting health care service
200: mobile terminal
300: health care service management server
310: membership management unit
320: questionnaire information provision and analysis unit
330: health record request and analysis unit
340: disease analysis unit
350: information provision and recommendation unit
360: online market management unit

## Claims

1. A system for supporting health care service, the system comprising:
at least one mobile terminal receiving an online health questionnaire including basic information, lifestyle, physical symptoms and interests using a web page, app page, program or application related to a health care service, providing questionnaire answers to the online health questionnaire, and and receiving search and recommendation for health status results analyzed based on the questionnaire answers and types of health functional foods and nutritional ingredients suitable for or helpful thereto; and
a health care service management server providing the online health questionnaire with question types consisting of basic information, lifestyle, symptoms and interests to the mobile terminal, collecting the questionnaire answers to the health questionnaire including single-choice, multi-choice entered on the mobile terminal,
analyzing disease information and diseases according to lifestyle and symptoms included in the collected questionnaire answers, and recommending nutritional ingredients suitable for the analysis results and providing recommendation information and sales information on health functional foods containing the recommended nutritional ingredients.

2. The system of claim 1,
wherein the health care service management server provides an interface that supports searching/writing/editing/deleting functions for health-related posts and SNS posts written by general and expert members and a comment function for posts and SNS posts; and
an interface that supports functions for sharing posts and SNS posts searched or written by general and expert members with other members.

3. The system of claim 2,
wherein the health care service management server provides an interface that supports search tab functions for disease (①), department (②), and health function (③),
the search tab for disease (①) supports the function of searching for information on definition, cause, related functionality, related food, TIP, and TALK, and
the search tab for department (②) supports the function of searching for related departments by keywords for age, main symptom, and side symptom, and
supports the function of displaying at least one department searched in order of proximity to the user on an electronic map through a location-based service.

4. The system of claim 3,
wherein the tab for the health function (③) supports the function of searching for and receiving recommendations for disease factors including nervous system, digestive and metabolic system, reproductive system, muscular system, sensory system, cardiovascular system, endocrine system, and immune system and
one or more of 1) detailed disease name, 2) nutritional ingredients or medicinal ingredients for preventing or suppressing the searched disease, 3) types of health functional foods containing the nutritional ingredients, 4) health habit information, and 5) sales information related thereto.

5. The system of claim 1,
wherein the health care service management server receives the user's health record information from the National Health Insurance Service under the Ministry of Health and Welfare,
classifies the medication information, medical history, and vaccination in the user's health record information, and
analyzes and provides, based on the medication information, medical history, and vaccination, at least one disease of interest information related thereto.

6. A method for supporting a health care service, the method comprising:
checking and providing, by a mobile terminal, health questionnaire answers to questions including basic information, lifestyle, physical symptoms provided by a health questionnaire platform;
analyzing and providing, by a health care service management server, disease types and categories according to the user's health status based on the health questionnaire answers;
recommending sales information and/or affiliated online shop sales information of preventive compositions effective for the disease types and categories analyzed by the health care service management server and health functional foods containing the preventive compositions; and
requesting, by the user, purchase, payment, and delivery processing of health functional foods that the user searched for on the mobile terminal or received the recommendation from the health care service management server,
requesting, by the health care service management server, when the product is delivered, a purchase review to be written to the mobile terminal, collecting the written purchase reviews and organizing the collected written purchase reviews into a database by member and product, and sharing the DB information with members.
